## Europäisches Patentamt

## European Patent Office

## Office européen des brevets

(19)

(11) Veröffentlichungsnummer: **0 016 388**
**B1**

(12) **EUROPÄISCHE PATENTSCHRIFT**

(45) Veröffentlichungstag der Patentschrift:
**12.01.83**

(51) Int. Cl.³: **C 07 C 113/04** // D06P3/68

(21) Anmeldenummer: **80101190.9**

(22) Anmeldetag: **10.03.80**

(54) **Verfahren zur Herstellung von Diazoniumsalzlösungen.**

(30) Priorität: **15.03.79 DE 2910199**

(43) Veröffentlichungstag der Anmeldung:
**01.10.80 Patentblatt 80/20**

(45) Bekanntmachung des Hinweises auf die Patenterteilung:
**12.01.83 Patentblatt 83/2**

(84) Benannte Vertragsstaaten:
**BE CH DE FR**

(56) Entgegenhaltungen:

**DE-A-2 334 769**
**DE-A-2 555 515**
**DE-B-1 023 771**
**Ullmanns Encyklopädie der technischen Chemie, 4.**
**Auflage, Band 8 (1974), Seite 248, linke Spalte,**
**Abs. d), Verlag Chemie, Weinheim/Bergstr.**

**Die Akte enthält technische Angaben, die nach dem**
**Eingang der Anmeldung eingereicht wurden und die**
**nicht in dieser Patentschrift enthalten sind.**

(73) Patentinhaber: **HOECHST AKTIENGESELLSCHAFT,**
**Postfach 80 03 20, D-6230 Frankfurt/Main 80 (DE)**

(72) Erfinder: **Feess, Erich Dr., Jahnstrasse 32,**
**D-6238 Hofheim Am Taunus (DE)**
Erfinder: **Hertel, Hasso Dr., Brunnenweg 10,**
**D-6052 Mühlheim/Main (DE)**

## Verfahren zur Herstellung von Diazoniumsalzlösungen

Die vorliegende Erfindung liegt auf dem Gebiet der Vorprodukte für Azofarbstoffe, insbesondere für die Herstellung von wasserunlöslichen Azofarbstoffen auf der Faser gemäss der Eisfarbentechnik.

Das Prinzip des Färbens mit Eintwicklungsfarbstoffen besteht in der Reaktion einer Kupplungskomponente mit einer Diazoniumverbindung der aromatischen Reihe, die keine wasserlöslich-machenden Gruppen enthält, auf der Faser. Obwohl schon seit vielen Jahren zur Arbeitserleichterung solche stabilisierten und eingestellten festen Diazoniumsalze im Handel sind, wird ein erheblicher Teil der verwendeten Diazoniumverbindungen vom Färber selbst durch Diazotierung der entsprechenden aromatischen Amine bereitet. Diese Diazotierung erfolgt, wie auch sonst in der Technik üblich (s. Ullmanns Encyklopädie der technischen Chemie, 4. Auflage, Band 8, Seite 246), mittels starker Säuren, so beispielsweise mit verdünnter Salzsäure. Die so erhaltenen Diazoniumsalzlösungen sind aber nicht nach blossem Verdünnen mit Wasser auf die in der Eisfarbentechnik erforderliche Konzentration als Entwicklungsflotten, z. B. in der Kontinuefärberei, anwendbar, sondern müssen zuvor durch Abstumpfen auf den hierfür erforderlichen höheren pH-Wert, der bei Kontinuefärbungen bei 2,5 bis 4,0 liegt, eingestellt werden.

Versucht man, die Diazotierung nicht mit starken Säuren, wie Salzsäure, auszuführen, sondern mit einer schwächeren Säure mit einem $pK_a$-Wert von etwa 2,5 bis 4,0, so entstehen Diazoniumsalzlösungen, die keiner pH-Anhebung bedürfen. Diese Methode der Diazotierung liefert jedoch für eine spätere Anwendbarkeit der erhaltenen Diazoniumsalzlösungen in der Eisfarbentechnik keine zufriedenstellenden Ergebnisse, da diese Lösungen Rückstände enthalten und keine ausreichende Haltbarkeit besitzen.

Zwar kann man gemäss der deutschen Patentschrift 1 023 771 in Wasser schwer lösliche primäre aromatische Amine, deren Salze in Wasser leicht hydrolysieren, bei einem pH-Wert von 2,9 bis 4 diazotieren, wenn sie in feiner Verteilung mit einer Teilchengrösse von kleiner als 30 µm, bevorzugt kleiner als 10 µm, und in Gegenwart eines Dispergiermittels vorliegen. Die Diazotierung ist jedoch auf diejenigen Amine beschränkt, die sich in eine solche feine Verteilung bringen lassen und deren Feinverteilung auch ausreichend lange stabil ist. Erfahrungsgemäss sind dies aber nur solche Amine, die einen Schmelzpunkt von grösser als etwa 70°C besitzen. Primäre aromatische Amine, die keine wasserlöslich-machenden Gruppen enthalten und die einen tieferen Schmelzpunkt haben, ergeben keine für die Anwendung in der Diazotierung über längerer Zeit stabil bleibende feinverteilte Dispersion oder Emulsion in Wasser.

Eine grosse Anzahl für die Eisfarbentechnik wichtiger primärer aromatischer Amine ist aber bei Raumtemperatur flüssig oder schmilzt nur wenig darüber, so beispielsweise das 2-Chloranilin, 3-Chloranilin, 3,5-Bis-(trifluormethyl)-anilin, 2-Chlor-5-trifluormethyl-anilin, 4-Chlor-2-trifluormethyl-anilin, 6-Chlor-2-amino-1-methyl-benzol, 5-Chlor-2-amino-1-methyl-benzol, 4-Chlor-2--amino-1-methylbenzol, 2-Amino-1-methoxy-benzol und das 5-Chlor-2-phenoxy-anilin. Nach dem bisherigen Stand der Technik sind diese nur mit verdünnten Mineralsäuren diazotierbar.

Bei der Diazotierung solcher niedrig schmelzenden aromatischen Amine gemäss der Verfahrensweise der deutschen Patentschrift 1 023 771, bei welcher die Amine in Dispersion und nicht in Lösung zur Diazotierung eingesetzt werden sollen, bilden sich allerdings erhebliche Mengen an unlöslichen Rückständen, die in Färbeprozessen der Eisfarbentechnik nicht toleriert werden können, da sie zu ungleichen Färbungen der Textilmaterialien führen. Zudem benötigt die Diazotierung selbst nach diesem bekannten Verfahren etwa 30 Minuten.

In der deutschen Offenlegungsschrift 23 34 769 wird erwähnt, nichtionogene Tenside als Dispergatoren zur Stabilisierung von Diazoniumsalzen zu verwenden. Doch selbst, wenn man solche Tenside in das Verfahren der deutschen Patentschrift 1 023 771 einsetzt, liefert dieses kombinierte Verfahren immer noch unbefriedigende Ergebnisse bei der Diazotierung, bei welcher ebenfalls unlösliche Rückstände anfallen. Auch das Verfahren der deutschen Offenlegungsschrift 25 55 515 löst nicht das Problem, — das im übrigen auch dort nicht zur Lösung anstand —, rasch und rückstandsfrei Diazoniumsalzlösung von primären aromatischen Aminen ohne wasserlöslich-machende Gruppe für eine einwandfreie Verwendung in der Eisfarbenfärberei einzusetzen.

Ergänzend seien noch Arbeiten von H. J. Ridd (s. z. B. J. Chem. Soc. 1958, 65) erwähnt, aus denen man zwar weiss, dass primäre aromatische Amine noch bei einem pH-Wert von 5,5 bis 5 quantitativ diazotiert werden können; allerdings gilt dies nur für die bei diesen kinetischen Versuchen angewendeten notwendigen sehr geringen Konzentrationen an dem Amin und der dabei erreichten Lösung aller Reaktionspartner.

Es bestand somit die Aufgabe, ein Diazotierungsverfahren zu finden, mit dem Diazoniumsalzlösungen von primären aromatischen Aminen, die keine wasserlöslich-machenden Gruppen enthalten, erhalten werden mit dem Ziel, dass diese Lösungen leicht in der Eisfarbenfärberei eingesetzt werden können. Durch die nachfolgend beschriebene Lösung dieser Aufgabe kann man solche in der Eisfarbentechnik verwendbaren primären aromatischen Amine mit einem Schmelzpunkt von 85°C oder kleiner als 85°C mit mittelstarken organischen Säuren mit einem pK$_a$-Wert von 2,5 bis 4,0 unter praxisüblichen Bedingungen diazotieren und erhält dabei Diazoniumsalzlösungen, die man direkt oder nach blosser Verdünnung mit Wasser oder verdünnter Säure ohne weitere pH-Einstellung zur Erzeugung von wasserunlöslichen Azofarbstoffen auf der Faser gemäss der Eisfarbentechnik verwenden kann.

Die vorliegende Erfindung betrifft somit ein Verfahren zur Herstellung von Diazoniumsalzlösungen aus primären aromatischen Aminen, die keine wasserlöslich-machende Gruppe enthalten, durch Diazotierung mittels eines Alkalinitrits, vorzugsweise Natriumnitrit, und einer mittelstarken organischen Säure, das dadurch gekennzeichnet ist, dass man die Lösung eines primären aromatischen Amins, das keine wasserlöslich-machende Gruppe enthält und einen Schmelzpunkt von 85°C oder niedriger als 85°C, vorzugsweise 70°C oder von unterhalb 70°C besitzt, in einem organischen nichtionogenen polaren oder unpolaren Lösungsmittel mit einem Gehalt an einem nichtionogenen Tensid mit Wasser verdünnt, das die nachgenannte organische Säure und gegebenenfalls bis zu 2 Mol Mineralsäure, wie Schwefelsäure, Phosphorsäure oder Salzsäure, zweckmässig Salzsäure, pro Mol Amin enthalten kann, und die Diazotierung mittels einer organischen Säure mit einem pK$_a$-Wert von 2,5 bis 4,0 in einer Menge von mindestens 2,2 Mol dieser organischen Säure pro Mol primärem aromatischem Amin und einem Alkalinitrit, vorzugsweise Natriumnitrit, durchführt.

Organische Säuren mit einem pK$_a$-Wert von 2,5 bis 4,0 sind beispielsweise Ameisensäure, Chloressigsäure, Milchsäure und Glykolsäure, des weiteren beispielsweise Dicarbonsäuren von Alkanen mit 2 bis 5 C-Atomen mit einem Gesamt-pK$_a$-Wert von 2,5 bis 4, der sich aus der Hälfte der Summe der beiden pK$_a$-Werte berechnet, wie z. B. Oxalsäure, Fumarsäure oder Weinsäure.

Nichtionogene Tenside, die erfindungsgemäss bei der Diazotierung anwesend sind, sind beispielsweise solche aus der Reihe der Polyglykoläther oder deren Derivate oder aus der Reihe der Zuckerderivate. Besonders geeignet sind beispielsweise Kondensationsprodukte aus phenolischen Verbindungen, wie dem Phenol selbst oder Kresol, Phenolpolyglykoläther und Formaldehyd, die auch nachmethyliert oder nachäthyliert sein können, des weiteren oxäthyliertes Ricinusöl, oxäthylierte Polypropylenglykoläther, oxäthylierte aliphatische Alkohole, oxäthylierte Phenole und oxäthylierte organische Phosphorsäureester. Solche nichtionogenen Tenside sind beispelsweise in K. Lindner, Tenside, Textilhilfsmittel-Waschrohstoffe, Band I, Stuttgart (1964), Seiten 837-962, beschrieben. Bevorzugt hiervon sind die folgenden zu nennen: Ricinusöl-Äthylenoxid-Addukte und Kondensationsprodukte aus Phenol, Phenolpolyglykoläther und Formaldehyd, letztere insbesondere als nachmethylierte Produkte, sowie Äthylenoxid-Addukte an einem Alkylphenol.

Organische Lösungsmittel, die erfindungsgemäss bevorzugt in den Lösungen der primären aromatischen Amine verwendet werden, sind Glykolverbindungen von (C$_1$-C$_4$)-Alkanen oder deren Di- bis Hexa-, insbesondere deren Di-, Tri- oder Tetrakondensationsprodukte, des weiteren deren Alkyläther, insbesondere deren niedere Alkyläther, ganz besonders deren niedere Monoalkyläther, so beispielsweise Äthylenglykol, Diäthylenglykol, Triäthylenglykol, Tetraäthylenglykol, Propylenglykol, Glykolmethyläther, Glykoläthyläther, Diäthylenglykol-monomethyl- oder -monoäthyläther, Diäthylenglykol-dimethyl- oder -diäthyläther, Tetraäthylglykol-monomethyl- oder -monoäthyläther, des weiteren N-Alkyl-carbonamid-Verbindungen, wie N-Methylpyrrolidon und Dimethylformamid, ebenso Dimethylsulfoxid. Als organische Lösungsmittel können aber auch die obengenannten nichtionogenen Tenside fungieren.

Für die Diazotierung des primären aromatischen Amins unter Verwendung von Alkalinitrit werden 2 Mol Säure benötigt. Zur Durchführung des erfindungsgemässen Verfahrens ist ein geringer Überschuss an Säure erforderlich, weswegen man die organische Säure in einer Menge von mindestens 2,2 Mol pro Mol Amin einsetzt. In der Regel werden in dem erfindungsgemässen Verfahren 2,2 bis 4 Mol der organischen Säure pro Mol primärem aromatischem Amin verwendet, doch ist ein höherer Überschuss an Säure für die nachfolgende Anwendung der Diazoniumsalzlösung als Entwicklungsflotte oder in der Entwicklungsstufe der Eisfarbenfärberei sogar erwünscht, da das Alkali aus dem mit der Kupplungskomponente alkalisch vorgrundierten Fasermaterial neutralisiert und das Reaktionsmedium der Entwicklungsstufe schwach sauer eingestellt werden muss. Im allgemeinen reicht die für die Azokupplung notwendige Bindung des Alkalis durch diesen Säureüberschuss aber nicht aus. Deshalb kann entweder von Anbeginn bei der Diazotierung mehr Säure, d. h. mehr als 4 Mol, eingesetzt werden, oder man gibt der erfindungsgemäss hergestellten Diazoniumsalzlösung anschliessend noch eine Säure zu.

Das erfindungsgemässe Verfahren wird vorteilhaft in der Weise durchgeführt, dass man eine Lösung des primären aromatischen Amins in dem organischen Lösungsmittel — oder in einer Mischung dieser Lösungsmittel — mit einem nichtionogenen Tensid oder einer Mischung derselben mit einer wässrigen Lösung der organischen Säure mit einem pK$_a$-Wert von 2,5 bis 4,0 vermischt, wobei man die Verdünnung auf das Diazotierungsvolumen vorteilhaft so wählt, dass das Amin bereits in einer solchen Konzentration vorliegt, dass die damit erhältliche Diazoniumsalzlösung direkt als Entwicklungsflotte oder in das Entwicklungsbad für die Eisfarbenfärberei eingesetzt werden kann. Die Diazotierung des primären aromatischen Amins, das nach der Verdünnung der organischen Lösung entweder weiterhin in Lösung vorliegt oder auch in einer feinverteilten stabilen Dispersion oder Emulsion vorliegen kann, erfolgt sodann durch Zugabe von festem Alkalinitrit oder vorzugsweise einer wässrigen Lösung eines Alkalinitrits. Die Diazotierung verläuft problemlos unabhängig von dem Grad der Verdünnung. Die erhaltene Diazoniumsalzlösung wird später, wie oben erwähnt, durch Verdünnen mit Wasser, das gegebenenfalls noch Säure enthält, auf die erforderliche Konzentration an dem Diazoniumsalz für die Entwicklung des Farbstoffes eingestellt.

Bei der Diazotierungsreaktion selbst ist eine Kühlung, insbesondere durch Verwendung von Eis, im Gegensatz zu den in der Eisfarbentechnik üblichen Diazotierungsmethoden nicht erforderlich.

Die nach dem erfindungsgemässen Verfahren hergestellten Diazoniumsalzlösungen werden in üblicher Weise, wie für die Eisfarbentechnik bekannt, als Entwicklungsflotten oder in die Bäder oder Flotten für die Entwickelungsstufe eingesetzt. Anwendungsvarianten sind in den Beispielen beschrieben. Mit dem erfindungsgemässen Verfahren lassen sich demnach leicht Diazoniumsalzlösungen auch von solchen primären aromatischen Aminen mit einem Schmelzpunkt von 85°C oder niedriger, insbesondere von 70°C oder unterhalb von 70°C, beispielsweise von Halogenanilinen, Halogentoluidinen oder Halogenanisidinen, herstellen und somit problemlos einer Anwendung in der Eisfarbenfärberei zuführen. Mit den erfindungsgemäss erhältlichen Diazoniumsalzlösungen werden hochwertige Färbungen von Azofarbstoffen auf der Faser mit sehr guten Echtheiten erhalten.

Die nachfolgenden Beispiele dienen zur Erläuterung der Erfindung; die Teile sind Gewichtsteile, die Prozentangaben beziehen sich auf Gewichtsprozente, sofern nicht anders vermerkt. Gewichtsteile verhalten sich zu Volumenteilen wie Kilogramm zum Liter.

Beispiel 1

620 Teile einer Lösung aus 600 Teilen 6-Chlor-2-amino-1-methyl-benzol, 350 Teilen eines Kondensationsproduktes aus Ricinusöl und 36 Mol Äthylenoxid, 25 Teilen Diglykol und 25 Teilen Methyltetraglykol, die durch einfaches Verrühren dieser Komponenten hergestellt wurde, werden in ein Gemisch aus 1030 Teilen Chloressigsäure und 10000 Teilen Wasser von 15°C eingerührt. Danach wird unter gutem Rühren eine Lösung aus 210 Teilen Natriumnitrit und 420 Teilen Wasser zugegeben. Nach etwa einer halben Minute ist die Diazotierung beendet und eine rückstandsfreie Lösung mit einem pH-Wert von 2,8 entstanden.

Anwendung

Zur Herstellung einer gelbstichigen Rotfärbung wird ein gebleichtes und mercerisiertes Baumwollgewebe auf einem Foulard mit einer heissen Grundierungsflotte geklotzt, die durch Eintragen von 20 Teilen 2-Hydroxy-naphthalin-3-carbonsäure-phenylamid in eine heisse Lösung von 27 Teilen 38%iger Natronlauge und 10 Teilen eines Eiweissabbauprodukt-Fettsäure-Kondensats in 950 Teilen heissem Wasser und Lösen der Kupplungskomponente durch Aufkochen hergestellt wird. Die Flottenaufnahme der geklotzten Ware beträgt 80% des Warengewichtes. Nach einem dem Klotzen folgenden kurzen Luftgang wird die Ware getrocknet und anschliessend mit der obigen Diazoniumsalzlösung geklotzt, die zuvor noch durch Zugabe von weiterem Wasser von 15°C und von 190 Teilen wässriger 60%iger Essigsäure auf 28500 Teile aufgefüllt worden war. Die Flottenaufnahme für die verdünnte Diazoniumsalzlösung beträgt ebenfalls 80% des Warengewichtes. Nach dem Aufbringen der Diazoniumsalzlösung auf die Ware folgt ein Luftgang und eine Heisswasserpassage; danach wird die Ware wie üblich gewaschen und getrocknet.

Die so erhaltene Färbung besitzt hohe Echtheitseigenschaften, wie beispielsweise Wasch-, Mercerisier- und Chlorechtheit.

Beispiel 2

Eine Lösung aus 500 Teilen 3-Chlor-anilin und 500 Teilen eines Ricinusöl-Äthylenoxid-Kondensats wird in 18000 Teile Wasser von 16°C eingerührt. Unter gutem Rühren gibt man nacheinander 850 Teile einer 85%igen wässrigen Ameisensäure und 1550 Teile einer wässrigen 20%igen Natriumnitritlösung hinzu. Nach etwa einer Minute ist die Diazotierung beendet, und man erhält eine rückstandsfreie Diazoniumsalzlösung, deren pH-Wert 3,6 beträgt.

Anwendung

Zur Herstellung einer orangen Färbung auf einem Textilmaterial aus Cellulosefasern verdünnt man diese Diazoniumsalzlösung mit Wasser auf das 2,3fache Volumen und setzt dabei noch 11 Teile einer 85%igen wässrigen Ameisensäure pro 1000 Teile dieser mit Wasser verdünnten Diazoniumsalzlösung hinzu. Die so erhaltene Entwicklungsflotte wird in das Chassis eines Foulards gegeben, und das Textilmaterial, das zuvor auf einem anderen Foulard mit einer heissen Lösung aus 20 Teilen 2-Hydroxynaphthalin-3-carbonsäure-5'-chlor-2'-methyl-phenylamid, 40 Teilen 33%iger Natronlauge sowie 10 Teilen eines Tensids auf Basis eines Fettsäure-Eiweissabbau-Kondensats in 1000 Teilen Wasser imprägniert und danach getrocknet wurde, wird durch diese Entwicklungsflotte hindurchgeführt. Nach einem Luftgang und einer Heisswasserpassage wird die gefärbte Ware in üblicher Weise fertiggestellt. (Die Flottenaufnahme sowohl bei der Grundierung als auch bei der Entwicklung beträgt 70% des Warengewichtes.) Diese orange Färbung zeigt ebenso sehr gute Echtheitseigenschaften, insbesondere Licht- und Waschechtheit.

**Beispiel 3**

Zur Durchführung des erfindungsgemässen Verfahrens verfährt man wie in Beispiel 2 beschrieben, verwendet jedoch anstelle des 3-Chlor-anilins 480 Teile o-Anisidin und anstelle des Tensids und organischen Lösungsmittels ein Gemisch aus 150 Teilen eines nachmethylierten Phenol-Phenolpolyglykoläther-Formaldehyd-Kondensats und 350 Teilen Methyldiglykol.

Die Anwendung der so erfindungsgemäss erhaltenen Diazoniumsalzlösung kann gemäss Beispiel 2 erfolgen. Es wird bei Einsatz der in Beispiel 2 genannten Kupplungskomponente eine blaustichig rote Färbung mit sehr guten Echtheitseigenschaften erhalten.

**Beispiel 4**

1000 Teile einer Lösung aus 600 Teilen 3-Chlor-anilin, 150 Teilen Diäthylenglykol-monomethyläther und 250 Teilen eines Kondensationsproduktes aus Ricinusöl und 36 Mol Äthylenoxid werden in ein Gemisch aus 820 Teilen 32%iger wässriger Salzsäure, 630 Teilen 85%iger wässriger Ameisensäure und 10000 Teilen Wasser von 20°C eingetragen. Unter Rühren werden sodann 350 Teile Natriumnitrit, in wenig Wasser gelöst, zugesetzt. Die Diazotierung ist innerhalb einer Minute beendet. Es wird eine rückstandsfreie Diazoniumsalzlösung erhalten.

Anstelle der Verwendung der organischen Säure (Ameisensäure) in dem in diesem Beispiel beschriebenen erfindungsgemässen Verfahren können auch als organische Säure 1050 Teile Milchsäure eingesetzt werden; desgleichen kann das nichtionogene Tensid (Ricinusöl-Äthylenoxid-Addukt) durch ein Phenol-Phenolpolyglykoläther-Formeldehyd-Kondensat als nichtionogenes Tensid ausgetauscht werden. Man erhält in jedem Falle eine rückstandsfreie Diazoniumsalzlösung, deren Diazotierungsreaktion ebenso schnell vonstatten geht.

Setzt man eine dieser drei Diazoniumsalzlösungen in analoger Weise zu der in Beispiel 1 beschriebenen Verfahrensweise zur Herstellung einer Färbung mit der im Beispiel 1 verwendeten Kupplungskomponente ein, so erhält man eine farbstarke orange Färbung mit guten Echtheitseigenschaften.

**Beispiel 5**

1000 Teile einer Lösung aus 600 Teilen 2-Chlor-anilin, 150 Teilen Diäthylenglykol-monoäthyläther und 250 Teilen eines Kondensationsproduktes aus Ricinusöl und 36 Mol Äthylenoxid werden in ein Gemisch aus 820 Teilen 32%-iger wässriger Salzsäure, 1120 Teilen Glykolsäure und 10000 Teilen Wasser von 20°C eingetragen. Unter Rühren werden sodann 375 Teile Natriumnitrit, in wenig Wasser gelöst, zugegeben. Innerhalb einer Minute ist die Diazotierungsreaktion beendet, und man erhält eine rückstandsfreie Diazoniumsalzlösung.

Anwendung

Zur Herstellung einer Färbung wird die Diazolösung mit Wasser von 20°C auf 50000 Teile verdünnt. Mit dieser fertigen Entwicklungsflotte verfährt man wie in Beispiel 1 beschrieben unter Anwendung der dort angegebenen Kupplungskomponente. Man erhält auf diese Weise eine scharlachfarbene Färbung mit sehr guten Echtheitseigenschaften.

**Beispiel 6**

Man verfährt gemäss dem in Beispiel 5 beschriebenen erfindungsgemässen Verfahren zur Herstellung einer Diazoniumsalzlösung, geht jedoch von einer organischen Lösung des 2-Chlor-anilins aus, die aus 600 Teilen 2-Chlor-anilin, 140 Teilen Diäthylenglykol-monoäthyläther, 150 Teilen Triäthylenglykol-monomethyläther und 100 Teilen eines Kondensationsproduktes aus Oleylalkohol und 23 Mol Äthylenoxid sowie aus 10 Teilen einer 50%igen Lösung von Triisobutylphosphat in Isobutanol besteht. Man erhält erfindungsgemäss eine rückstandsfreie Lösung des Diazoniumsalzes des 2-Chlor-anilins.

Diese Diazoniumsalzlösung kann man gemäss den Angaben des Beispieles 5 zur Herstellung einer Färbung gemäss der Eisfarbentechnik einsetzen und erhält ebenfalls eine scharlachfarbene Färbung mit guten Echtheiten.

**Beispiel 7**

1000 Teile einer Lösung aus 715 Teilen 5-Chlor-2-amino-toluol, 180 Teilen Dimethylformamid und 105 Teilen eines Kondensationsproduktes aus Ricinusöl und 36 Mol Äthylenoxid werden in ein Gemisch aus 880 Teilen 32%iger wässriger Salzsäure, 800 Teilen 85%iger Ameisensäure und 10000 Teilen Wasser von 20°C unter Rühren eingetragen. Sodann werden unter weiterem Rühren 365 Teile Natriumnitrit, in wenig Wasser gelöst, zugegeben. Die Diazotierungsreaktion ist innerhalb einer Minuten beendet, und man erhält eine rückstandsfreie Diazoniumsalzlösung.

Anwendung

Man verdünnt diese Diazoniumsalzlösung mit Wasser von 20°C auf 50000 Teile und verfährt im übrigen zur Herstellung einer Färbung auf Baumwolle gemäss der Verfahrensweise des Beispieles 1 unter Verwendung der dort angegebenen Kupplungskomponente. Man erhält eine tiefe Rotfärbung mit guten Echtheitseigenschaften.

**Beispiel 8**

In gleicher Weise wie im Beispiel 7 beschrieben lässt sich eine rückstandslose Diazoniumsalzlösung des 5-Chlor-2-amino-toluols herstellen, wenn man anstelle der im Beispiel 7 beschriebenen organischen Lösung dieses Amins von einer Lösung aus 715 Teilen 5-Chlor-2-amino-toluol, 200 Teilen Dimethylsulfoxid und 85 Teilen eines Kondensationsproduktes aus Nonylphenol und 30 Mol Äthylenoxid — oder anstelle dieses Nonylphenol-Äthylenoxid-Adduktes 85 Teile eines Kondensationsproduktes aus Laurylalkohol und 10 Mol Äthylenoxid — ausgeht.

Mit dieser Diazoniumsalzlösung lassen sich ebenfalls kräftige Färbungen mit guten Echtheitseigenschaften herstellen, so beispielsweise bei Anwendung der Kupplungskomponente des Beispieles 7 eine rote Färbung.

Beispiel 9

1000 Teile einer Lösung aus 600 Teilen 4-Chlor-2-amino-toluol, 150 Teilen N-Methyl-pyrrolidon und 250 Teilen eines Kondensationsproduktes aus Ricinusöl und 40 Mol Äthylenoxid werden in ein Gemisch aus 500 Teilen 32,5%-iger wässriger Salzsäure, 970 Teilen Glykolsäure und 10000 Teilen Wasser von 20°C eingetragen. Unter weiterem Rühren gibt man 340 Teile Natriumnitrit, in wenig Wasser gelöst, zu. Innerhalb einer Reaktionszeit von einer Minute ist die Diazotierung beendet. Man erhält eine rückstandsfreie, klare Diazoniumsalzlösung.

Anwendung Zur Anwendung als Entwicklungsflotte wird diese Diazoniumsalzlösung durch Zugabe von weiterem Wasser von 20°C und von 100 Teilen einer 85%igen wässrigen Ameisensäure auf 45000 Teile aufgefüllt. Die Durchführung der Färbung erfolgt sodann gemäss der in Beispiel 1 beschriebenen Verfahrensweise. Man erhält unter Anwendung der in Beispiel 1 eingesetzten Kupplungskomponente eine kräftige Rotfärbung mit guten Echtheiten.

Beispiel 10

1000 Teile einer Lösung aus 500 Teilen 4-Chlor-2-amino-anisol, 400 Teilen N-Methyl-pyrrolidon und 100 Teilen eines Kondensationsproduktes aus Ricinusöl und 36 Mol Äthylenoxid werden in ein Gemisch aus 550 Teilen 32%iger wässriger Salzsäure, 520 Teilen 85%iger wässriger Ameisensäure und 31000 Teilen Wasser von 20°C eingetragen. Unter weiterem Rühren werden 250 Teile Natriumnitrit, in wenig Wasser gelöst, zugegeben. Die Diazotierungsreaktion ist innerhalb einer Minute beendet. Es wird eine rückstandsfreie, klare Diazoniumsalzlösung erhalten.

Zur Herstellung einer roten Färbung auf Cellulosefasermaterial mit guten Echtheiten kann man gemäss der Verfahrensweise des Beispieles 1 die so hergestellte Diazoniumsalzlösung als Entwicklungsflotte einsetzen.

Beispiel 11

Man verfährt wie in Beispiel 10 in erfindungsgemässer Weise zur Herstellung einer Diazoniumsalzlösung, verwendet jedoch als nichtionogenes Tensid anstelle des Ricinusöl-Äthylenoxid-Adduktes ein Polymerisationsprodukt aus Propylenoxid und Äthylenoxid mit einem Gehalt von 80% Äthylenoxid. Man erhält in gleicher Weise eine klare Diazoniumsalzlösung, die ebenso, wie in Beispiel 10 beschrieben, eine farbstarke rote Färbung mit guten Echtheiten ergibt.

**Patentansprüche**

1. Verfahren zur Herstellung von Diazoniumsalzlösungen aus primären aromatischen Aminen, die keine wasserlöslich-machende Gruppe enthalten, durch Diazotierung mittels eines Alkalinitrits und einer mittelstarken organischen Säure, dadurch gekennzeichnet, dass man die Lösung eines primären aromatischen Amins, das keine wasserlöslich-machende Gruppe enthält und einen Schmelzpunkt von 85°C oder niedriger besitzt, in einem organischen, nicht-ionogenen polaren oder unpolaren Lösungsmittel mit einem Gehalt an einem nicht-ionogenen Tensid mit Wasser verdünnt, das die nachgenannte organische Säure und gegebenenfalls bis zu 2 Mol Mineralsäure pro Mol Amin enthalten kann, und die Diazotierung mittels einer organischen Säure mit einem pK$_a$-Wert von 2,5 bis 4,0 in einer Menge von mindestens 2,2 Mol dieser organischen Säure pro Mol primärem aromatischem Amin und einem Alkalinitrit durchführt.

2. Verfahren nach Anspruch 1, dadurch gekennzeichnet, dass das primäre aromatische Amin einen Schmelzpunkt von 70°C oder von unterhalb 70°C besitzt.

3. Verfahren nach Anspruch 1 oder 2, dadurch gekennzeichnet, dass die organische Säure Ameisensäure, Chloressigsäure, Milchsäure, Glykolsäure, Oxalsäure, Fumarsäure oder Weinsäure ist.

4. Verfahren nach Anspruch 1, 2 oder 3, dadurch gekennzeichnet, dass das nicht-ionogene Tensid ein Ricinusöl-Äthylenoxid-Addukt oder ein Phenol-Phenolpolyglykoläther-Formaldehyd-Kondensationsprodukt, das nachmethyliert oder nachäthyliert sein kann, oder ein Äthylenoxidaddukt an einem Alkylphenol oder an einem aliphatischen Alkohol ist.

5. Verfahren nach Anspruch 1, 2, 3 oder 4, dadurch gekennzeichnet, dass das nicht-ionogene Lösungsmittel Äthylenglykol, Diäthylenglykol, Triäthylenglykol oder Tetraäthylenglykol oder deren niedere Monoalkyläther ist.

6. Verfahren nach Anspruch 1, 3, 4 oder 5, dadurch gekennzeichnet, dass das aromatische Amin ein Halogenanilin, Halogentoluidin oder Halogenanisidin ist.

7. Verfahren nach einem der Ansprüche 1 bis 6, dadurch gekennzeichnet, dass man die Lösung des primären aromatischen Amins in dem nicht-ionogenen Lösungsmittel und nicht-ionogenen Tensid erst mit Wasser, sodann mit der organischen Säure mit dem pK$_a$-Wert von 2,5 bis 4,0 und sodann mit einer wässrigen Lösung des Alkalinitrits zusammengibt.

**Claims**

1. Process for the manufacture of diazonium salt solutions of primary aromatic amines not containing a water-solubilizing group, by diazotization with the aid of an alkali nitrite and an organic acid of medium strength, characterized by that the solution of a primary aromatic amine not containing a water-solubilizing group and having a melting point of 85°C or below, is diluted in an organic, non-ionogenic polar or nonpolar solvent containing a non-ionogenic surfactant, with water which contains the organic acid mentioned below and optionally up to 2 mols of a mineral acid per mol amine, and the diazotization is carried out by means of an organic acid having a $pK_a$-value of from 2.5 to 4.0, in an amount of at least 2.2 mols of said organic acid per mol of primary aromatic amine, and by means of an alkali metal nitrite.

2. A process according to claim 1, characterized by that the primary aromatic amine has a melting point of 70°C or less than 70°C.

3. A process according to claim 1 or 2, characterized by that the organic acid is formic acid, chloroacetic acid, lactic acid, glycolic acid, oxalic acid, fumaric acid or tartaric acid.

4. A process according to claim 1, 2 or 3, characterized by that the non-ionogenic surfactant is a castor oil-ethylene oxide addition product or is a phenol-phenolpolyglycolether-formaldehyd condensation product which may be post-methylated or post-ethylated, or is an ethylene oxide addition product at an alkyl phenol or at an aliphatic alcohol.

5. A process according to claim 1, 2, 3 or 4, characterized by that the non-ionogenic solvent is ethylene glycol, diethylene glycol, triethylene glycol or tetraethylene glycol, or the lower monoalkyl ethers thereof.

6. A process according to claim 1, 3, 4 or 5, characterized by that the aromatic amine is a halogeno-aniline, a halogeno-toluidine or a halogeno-anisidine.

7. A process according to one of claims 1 to 6, characterized by that the solution of the primary aromatic amine in the non-ionogenic solvent and non-ionogenic surfactant is mixed first with water, then with the organic acid having the $pK_a$-value of from 2.5 to 4.0, and thereafter with an aqueous solution of the alkali metal nitrite.

**Revendications**

1. Procédé pour préparer des solutions de sel de diazonium à partir d'amines aromatiques primaires qui ne contiennent pas de groupes de solubilisation dans l'eau, par diazotation à l'aide d'un nitrite alcalin et d'un acide organique de force moyenne, procédé caractérisé en ce qu'on soumet la solution d'une amine aromatique primaire, qui ne contient pas de groupes de solubilisation dans l'eau et présente un point de fusion égal ou inférieur à 85°C, dans un solvant organique, non ionogène, polaire ou non polaire, et qui contient un tensioactif non ionogène, à une diluition avec de l'eau qui peut contenir l'acide organique indiqué ci-après et éventuellement jusqu'à 2 moles d'un acide minéral par mole d'amine, et l'on effectue la diazotation à l'aide d'un acide organique ayant un $pK_a$ de 2,5 à 4,0 et que l'on utilise en une quantité d'au moins 2,2 moles de cet acide organique par mole d'amine aromatique primaire et d'un nitrite alcalin.

2. Procédé selon la revendication 1, caractérisé en ce que l'amine aromatique primaire présente un point de fusion égale ou inférieur à 70°C.

3. Procédé selon l'une des revendications 1 et 2, caractérisé en ce que l'acide organique est l'acide formique, l'acide chloracétique, l'acide lactique, l'acide glycolique, l'acide oxalique, l'acide fumarique ou l'acide tartarique.

4. Procédé selon l'une quelconque des revendications 1, 2 ou 3, caractérisé en ce que le tensioactif non ionogène est un produit d'addition de l'oxyde d'éthylène sur l'huile de ricin ou un produit de condensation du phénol, d'un éther polyglycolique de phénol et du formaldéhyde qui peut avoir été ensuite méthylé ou éthylé, ou un produit d'addition de l'oxyde d'éthylène sur un alkyl-phénol ou sur un alcool aliphatique.

5. Procédé selon l'une quelconque des revendications 1 à 4, caractérisé en ce que le solvant non ionogène est l'éthylène-glycol, le diéthylène-glycol, le triéthylène-glycol ou le tétraéthylène-glycol ou un de leurs éthers monoalkyliques inférieurs.

6. Procédé selon l'une quelconque des revendications 1, 3, 4 ou 5, caractérisé en ce que l'amine aromatique est une halogéno-aniline, une halogéno-toluidine ou une halogéno-anisidine.

7. Procédé selon l'une quelconque des revendications 1 à 6, caractérisé en ce qu'on combine la solution de l'amine aromatique primaire dans le solvant non ionogène et le tensioactif non ionogène tout d'abord avec de l'eau, puis avec l'acide organique dont le $pK_a$ se situe entre 2,5 et 4,0, puis avec une solution aqueuse du nitrite alcalin.